# EUROPEAN PATENT APPLICATION

(11) **EP 3 659 572 A1**
(43) Date of publication of application: **03.06.2020**
(21) Application number: 18837250.2
(22) Date of filing: 09.07.2018
(51) Int. Cl.: A61H 9/00, A61H 7/00

(54) **METHOD AND APPARATUS FOR HAIR GROWTH/HAIR RESTORATION OPERATION**

(30) Priority: 28.07.2017 JP 2017147163
(71) Applicant: Hayashi, Kenji, Toyono-gun, Osaka 563-0132 (JP)
(72) Inventor: Hayashi, Kenji, Toyono-gun, Osaka 563-0132 (JP)
(74) Representative: Hauck Patentanwaltspartnerschaft mbB
(86) International application number: PCT/JP2018/025901
(87) International publication number: WO 2019/021798

(57) **Abstract**

The invention is a method for a hair growth/hair restration promoting treatment, characterized by comprising pinching a part of skin where hair growth/hair restoration is to be promoted and jetting a pressure fluid to the part from a periphery thereof to raise the part to burn fat under a skin layer in and near the part to which the pressure fluid is jetted to improve blood circulation to perform a treatment for promoting hair growth/hair restoration, and a hair growth/hair restoration promoting apparatus 1 characterized by comprising a pressure fluid supply source 2, a plurality of pressure fluid supply pipes 4 configured to allow a pressure fluid supplied from the pressure fluid supply source 2 to pass therethrough, a plurality of nozzles 5 formed on ends of the pressure fluid supply pipes 4 at sides facing each other, the nozzles being configured to jet the pressure fluid and nozzle separation/approach means 6 for causing the plurality of nozzles 5 of the plurality of pressure fluid supply pipes 4 to separate from and approach each other.

## Description

### TECHNICAL FIELD

The present invention relates to a hair growth/hair restoration treatment method for promoting hair growth or hair restoration using the jet pressure of a fluid and an apparatus used in the method.

### BACKGROUND ART

Patent Literature 1 discloses an apparatus that achieves hair growth/hair restoration by promoting blood circulation in skin without using an agent, such as a hair grower or a hair restorer, or frequency. This conventional technique is an apparatus that promotes hair growth or hair restoration in the following manner. A large-diameter opening of an attachment having an inverted funnel shape is put on a part of the head where hair growth is to be promoted, and outside air is drawn through an air flowing hole which is open on the peripheral side face of the attachment by suction from a cylindrical pipe on the upper end, thereby lifting the scalp in the part where hair growth is to be promoted by the suction force and applying a stimulus to improve blood circulation and feed nutrients to the root of hair.

A hair growth promoting apparatus disclosed in Patent Literature 2 induces hair growth by rotating a roller set inside a hat-like case by an electric motor mounted on the hat-like case and massaging the scalp with the roller.

### CITATION LIST

### PATENT LITERATURE

Patent Literature 1: JP2000-288048 A
Patent Literature 2: JP2001-190621 A

### SUMMARY OF THE INVENTION

### TECHNICAL PROBLEMS

However, according to the invention of Patent Literature 1, since the opening is put on the part of the head where hair growth is to be promoted, and the suction is performed from the cylindrical pipe, there is a problem in that hair is sucked into the cylindrical pipe together with air, which makes the suction difficult. Further, when shampoo is used for washing or when a hair restorer is used to promote hair restoration, the shampoo or the hair restorer is also sucked at the same time. Thus, a suction machine for both gas and liquid is required, which is disadvantageously uneconomical.

Further, according to the invention of Patent Literature 2, the massage is performed by pressing the roller, which is rotated by the electric motor fixed to the head with a belt, against the head. Thus, there is a problem in that, when hair is left on the head, the hair becomes entangled with the roller. Further, a part where the massage can be performed is limited to a rotary movable range of the roller. Thus, there is a problem in that, when a part where hair growth is to be promoted falls out of the movable range of the roller, no effect can be expected.

### SOLUTION TO PROBLEMS

The present patent application was filed sinse the applicant/the inventor got some sucsessful results leading to the invention to solve the said problems regarding the resurch of internal structure of epidermis of human body which becomes a factor for hair growth/hair restoration, method for hair growth/hair restoration treatment based on the resurch result and the invention of operation to embody the method.

The invention of claim 1 of the present application is a treatment method for promoting hair growth/hair restoration, characterized by comprising pinching a part of skin where hair growth/hair restoration is to be promoted and jetting a pressure fluid to the part from a periphery thereof to raise the part to burn fat under a skin layer in and near the part to which the pressure fluid is jetted to improve blood circulation to perform a treatment for promoting hair growth/hair restoration.

The treatment method for promoting hair growth/hair restoration, wherein the pressure fluid is air.

The treatment method for promoting hair growth/hair restoration, wherein the pressure fluid is water.

The invention of claim 4 of the present application relates to a apparatus for using the treatment method, wherein it is a hair growth/hair restoration promoting apparatus, characterized by comprising a pressure fluid supply source, a plurality of pressure fluid supply pipes configured to allow a pressure fluid supplied from the pressure fluid supply source to pass therethrough, a plurality of nozzles formed on ends of the pressure fluid supply pipes at sides facing each other, the nozzles being configured to jet the pressure fluid and nozzle separation/approach means for causing the plurality of nozzles of the plurality of pressure fluid supply pipes to separate from and approach each other.

The hair growth/hair restoration promoting apparatus, wherein the pressure fluid is air.

The hair growth/hair restoration promoting apparatus, wherein the pressure fluid is water.

The hair growth/hair restoration promoting apparatus, wherein the nozzle separation/approach means includes long slender pressure fluid supply pipes having a restoring force, the pressure fluid supply pipes being separable and approachable by an elastic force applied in an open direction of nozzles of the pressure fluid supply pipes or an opposite direction thereof.

The hair growth/hair restoration promoting apparatus, wherein the nozzles include an open hole open on a tip of a bent tip part of the pressure fluid supply pipe.

The hair growth/hair restoration promoting apparatus, wherein the nozzles include an open hole open on a side face of a tip part of the pressure fluid supply pipe.

The hair growth/hair restoration promoting apparatus, wherein the nozzles include a plurality of open holes open on a branch pipe disposed on a tip of the pressure fluid supply pipe so that the pressure fluid passes therethrough.

### ADVANTAGEOUS EFFECTS OF INVENTION

According to a hair growth/hair restoration promoting method of the present invention, the part of the skin layer where hair growth/hair restoration is to be promoted is pinched, and the pressure fluid is jetted to the part from the periphery thereof to raise the part to split coagulated fat cells present under the skin layer in or near the part to which the pressure fluid is jetted. Fat is burned by releasing the entanglement of the fat cells, and blood vessels which have been blocked by the coagulation of fat cells are opened to improve blood circulation, thereby promoting hair growth/hair restoration.

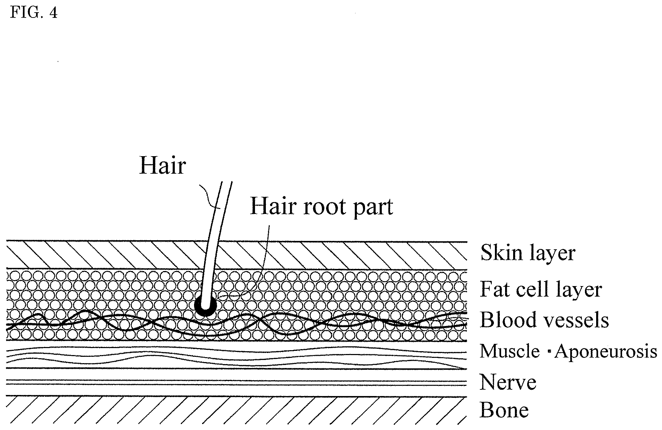

According to the knowledge of the applicant and the like, as illustrated in Fig. 4, the schematic skin structure of a human body includes a bone, a nerve, a muscle/aponeurosis layer, a blood layer including capillaries, a fat cell layer including a part of the blood vessel layer, and a skin layer on the outer side which are formed in this order from the inside toward the outside of the body. Further, a hair root part of hair including hair on the head is present in the fat cell layer including a part of the blood vessel layer. Under a knowledge that, in a part of the skin layer with thin hair or no hair on the head, blood circulation is not smooth due to fat cells coagulated in the fat cell layer of the skin structure, the applicant has reached a conclusion that it is necessary to open the blocked blood vessels by splitting the coagulated fat cells to release the entanglement of the fat cells, and has invented the above treatment method as a specific technique thereof.

Specifically, the skin layer in a part of the scalp with thin hair or no hair is pinched, and the pressure fluid supplied from the pressure fluid supply source, the pressure fluid not hurting the skin layer so that the skin layer can endure the pressure fluid, is jetted to the part from the periphery thereof, for example, from at least both sides or three or more sides. Accordingly, the skin layer of the pinched part forms a raised part in a state as pinched with fingers by the pressure fluid. The pressure fluid is continuously blown to the skin layer surface in the raised part. Thus, fat cells inside the skin layer are split by the pressure to release the entanglement of the fat cells, thereby burning fat and opening the blocked blood vessels. Accordingly, dull circulation of blood inside the blood vessels including capillaries inside the coagulated fat cells is improved, and nutrients of hair are supplied to the hair root part, which promotes hair growth or hair restoration.

When the pressure fluid is air, an existing apparatus which is easy to carry can be used as the pressure fluid supply source. Thus, the apparatus is less likely to have a limitation on a place where the treatment is performed and can thus be easily used. Further, an existing apparatus such as a compressor can also be used. Thus, the cost can be reduced.

When the pressure fluid is water, the scalp can be cleaned while performing the treatment.

The apparatus includes the nozzles which are formed on the ends of the pressure fluid supply pipes at the sides facing each other and jet the pressure fluid using the pressure fluid supply pipes. Thus, when the nozzles are put on a part of the head where hair growth/hair restoration is to be promoted, and the distance between the nozzles is reduced by the nozzle separation/approach means while jetting the pressure fluid from the nozzles, the skin layer is pushed up by the pressure fluid from both sides or the periphery thereof and brought into a raised state as pinched up with fingers. Accordingly, it is possible to split fat cells to release the entanglement of the fat cells with a short period of time. The treatment can be performed on the skin layer in a large area by sequentially moving the nozzles. When the pressure fluid is air or water, there are advantages as described above. Specifically, when the pressure fluid is air, an existing apparatus can be used, and the cost is thus low. When the pressure fluid is water, since the pressure of tap water is sufficient, a lower-cost apparatus can be used.

When the nozzle separation/approach means is long slender pressure fluid supply pipes having a restoring property, the part to be treated can be moved by alternately repeating gripping and loosening with fingers in such a manner that the pressure fluid supply pipes are gripped with the fingers when the nozzles on the ends of the pressure fluid supply pipes are caused to approach each other and loosened when the nozzles are caused to separate from each other. Thus, the part in a large area can be sequentially treated with one hand. Further, when the nozzle is an open hole open on the tip of the bent tip part of the pressure fluid supply pipe, a space is can be obtained between the bent tip having the open hole and the pressure fluid supply pipe. Thus, it is easy to pinch the part of the skin layer where hair growth/hair restoration is to be promoted. Further, the raised part and the pressure fluid supply pipe are not brought into contact with each other during the treatment, and the treatment can be smoothly performed. Further, when the nozzle is an open hole open on the side face of the tip part of the pressure supply pipe, the apparatus itself can be downsized. In addition, since hair is not caught on the tip part even in the treatment on a part with hair on the head, the efficiency of the treatment operation is improved. Further, when the nozzle is a plurality of open holes open on the branch pipe which is disposed on the tip of the pressure fluid supply pipe so that the pressure fluid passes therethrough, the treatment can be performed on a part having a large area on the head. Thus, the treatment efficiency is increased. When an attachment plate is provided, the operation can be made easy even when the number of pressure fluid supply pipes is increased.

### BRIEF DESCRIPTION OF THE DRAWINGS

Fig 1. is a front view of a main part in a first embodiment of a hair growth/hair restoration promoting apparatus.
Fig 2. is an example view of treatment use for a apparatus in Figs. 1.
Fig. 3 is a rear side example view of treatment use using a apparatus in Fig. 2.
Fig. 4 is a outline of sectional explanatory view of the skin layer of human body.
Fig. 5 is an enlarged explanatory view of a section of the skin layer A-A line illustrated in Fig. 2.
Fig .6 is a front view of a main part in a second embodiment of a hair growth/hair restoration promoting apparatus.
Fig. 7 is a front view of a main part in a third embodiment of a hair growth/hair restoration promoting apparatus.
Fig. 8 is an example view of main part of treatment use of an apparatus in Fig. 7.
Fig. 9 is an explanatory view of the nozzels in a forth embodiment of a hair growth/hair restoration promoting apparatus.
Fig. 10 is an example view of treatment use using the nozzels in Fig. 9
Fig. 11 is a perspective explanatory view in a fifth embodiment of a hair growth/hair restoration promoting apparatus.
Fig. 12 is a front view of a main part in a sixth embodiment of a hair growth/hair restoration promoting apparatus.
Fig. 13 is a side view of main part in Fig. 11.
Fig. 14 is a view showing the state of hair before the treatment and after the treatment (Case 1).
Fig. 15 a view showing the state of hair before the treatment and after the treatment (Case 2).

### DESCRIPTION OF EMBODIMENTS

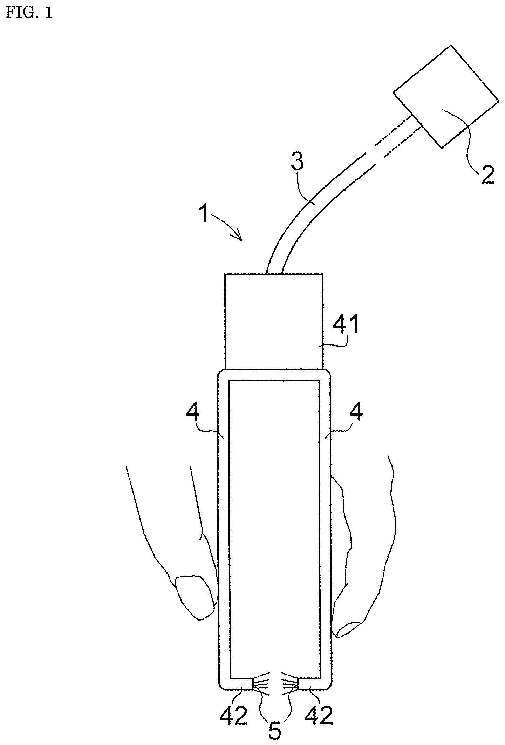

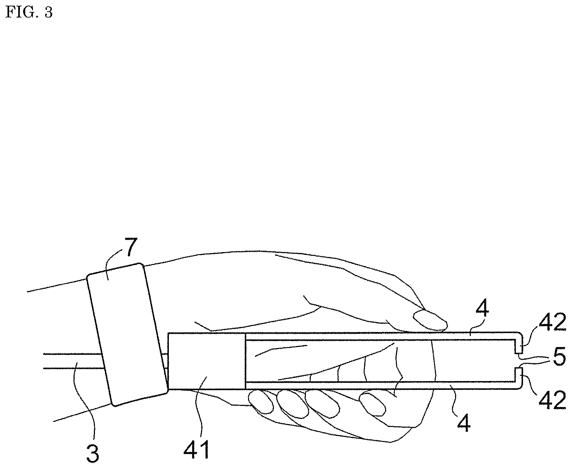

Embodiments of the present invention will be described with reference to the drawings. First, a first embodiment of a hair growth/hair restoration promoting apparatus 1 of the present invention will be described wit reference to Figs. 1 to 3. The hair growth/hair restoration promoting apparatus 1 of the present invention mainly includes a pressure fluid supply source 2, which includes, for example, a cylinder in which compressed air stored by an air compression pump or the like is stored, a plurality of pressure fluid supply pipes 4 to which a pressure fluid is supplied from the pressure fluid supply source 2 through a coupling pipe 3, a plurality of nozzle 5, which are disposed on ends of the pressure fluid supply pipes 4, and nozzle separation/approach means 6 for causing the nozzles 5 of the pressure fluid supply pipes 4 to separate from and approach each other.

When the pressure fluid to be supplied is gas such as air, the pressure fluid supply source 2 may directly supply gas from a compressor or the like or supply air compressed and stored in a cylinder or the like. When the pressure fluid is liquid such as water, the pressure fluid supply source 2 may directly supply liquid from a feed water pump or may be a tap water supply source when tap water is used. In all these cases, the pressure fluid supply source 2 is desirably a supply source capable of continuously supplying a pressure fluid at constant pressure.

The plurality of pressure fluid supply pipes 4 are used. Each of the pressure fluid supply pipes 4 includes a long slender metal pipe having a restoring force, and the nozzle 5 which jets the pressure fluid is disposed on the end thereof. In the embodiment illustrated in Fig. 1, two pressure fluid supply pipes 4 are used. One end of each of the pressure fluid supply pipes 4 is connected to a branch pipe 41, which is coupled to the coupling pipe 3, the other end thereof is open on the tip of a pinch pipe part 42, which perpendicularly bends toward the side facing the other pressure fluid supply pipe 4, and the nozzle 5 is open on the other end. Further, the two pressure fluid supply pipes 4 return to an original parallel state by the nozzle separation/approach means 6. In the present embodiment, the pressure fluid supply pipe 4 is a stainless pipe having a length of 100 mm and an outer diameter of 2.0 mm. The inner diameter of the nozzle 3 is 1.0 mm. The distance between the pressure fluid supply pipes 4 is 20 mm. The length of the pinch pipe part 42 is 5 mm. The distance between the tips of the nozzles 5 is 10 mm. The pressure fluid supply pipe 4 is not limited to the metal pipe having a restoring force, and may be an elastic hard synthetic resin pipe.

The nozzle separation/approach means 6 in the present embodiment is the long slender metal pipes having a restoring force as the pressure fluid supply pipes 4. When the distance between the tips is reduced by gripping the two pressure fluid supply pipes 4 with fingers, and the fingers are then released, the distance between the tips returns to its original distance by the restoring force of the metal pipes. A stainless steel pipe is suitable for the metal pipe having a restoring force.

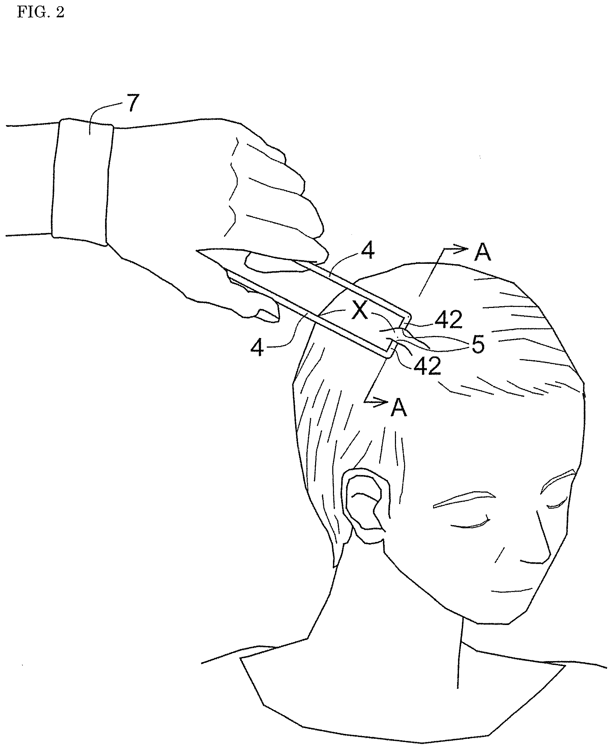

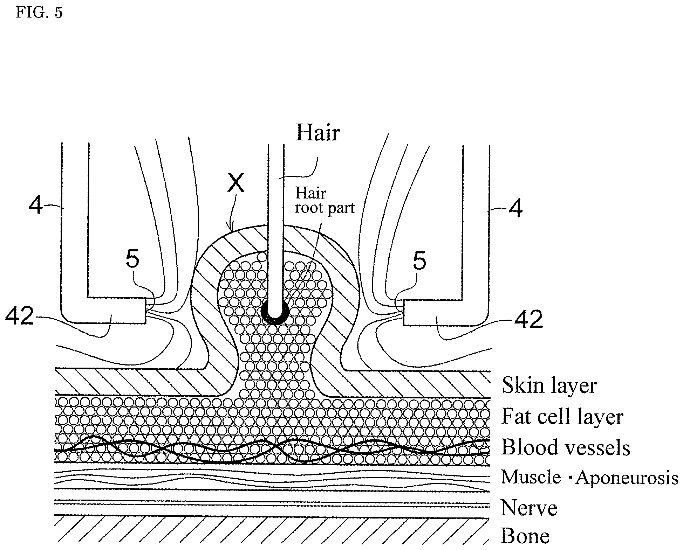

The use of the hair growth/hair restoration promoting apparatus 1 of the first embodiment having the above configuration will be described with reference to Figs. 1 to 5. As illustrated in Fig. 2, an operator pinches the two pressure fluid supply sources 2 of the hair growth/hair restoration promoting apparatus 1 between the thumb and forefinger, puts the branch pipe 41 on the back of the hand, and attaches the coupling pipe 3, which is connected to the pressure fluid supply source 2, to the wrist with a band 7. At the stage when such a preparation is completed, as illustrated in Fig. 2, the operator grips the two pressure fluid supply pipes 4 as the nozzle separation/approach means 6 in the direction for reducing the distance between the two nozzles 5 of the pressure fluid supply pipes 4 against the restoring force of the pressure fluid supply pipes 4 while lightly putting the nozzles 5 on a part of a skin layer of the head of a patient where hair growth/hair restoration is to be promoted. The pressure fluid from the pressure fluid supply source 2 is continuously jetted from the tips of the nozzles 5 through the coupling pipe 3 and the pressure fluid supply pipes 4. Thus, when the distance between the nozzles 5 is reduced and becomes narrow, as illustrated in Fig. 2 and Fig. 5, which is an enlarged sectional view, the air pressure is applied to the part of the skin layer from both sides thereof. Thus, the part is further lifted to form a raised part X.

This state will be described with reference to Fig. 5. The pressure fluid is continuously jetted to the raised part X of the skin layer, and the raised skin layer gradually becomes narrow. Accordingly, fat cells held and coagulated inside the raised skin layer are split into individual cells by the fluid pressure. The split of the fat cells burns nutrients stored between the cells, the flow of blood in blood vessels is improved along with the movement of muscles, and nutrients are fed to a hair root part. Alternatively, it is considered that a new hair root is generated from the skin layer or a fat cell layer, which promotes hair growth. While the same part of the skin layer is held with the nozzles 5 of the pressure fluid supply pipes 4, a split sound, such as a popping sound, caused by the split of fat cells is generated. The holding time is not particularly limited to any time, and may be approximately several seconds. Then, the gripping fingers are loosened to return the pressure fluid supply pipes 4 by the nozzle separation/approach means 6, thereby expanding the distance between the nozzles 5. Then, a move to a new part is made, and the same treatment is performed thereon. Alternatively, the nozzles 5 may be slid while maintaining the gripping state to make a move to another continuous part. Such a treatment is performed on a part where hair growth/hair restoration is to be promoted. Thus, the treatment time increases in proportion to the treatment area. In the present embodiment, the operator performs the treatment on the patient. However, the patient himself/herself can also perform the treatment while looking in a mirror as needed.

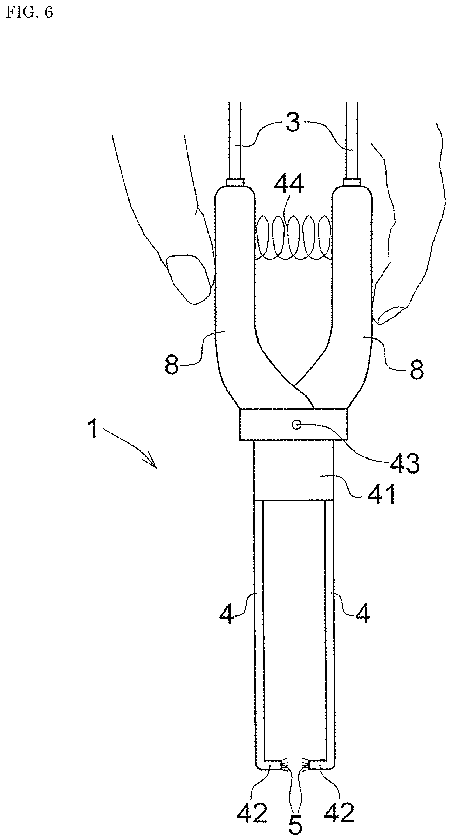

Fig. 6 illustrates a second embodiment of the present invention. The second embodiment differs from the first embodiment in that nozzle separation/approach means 6 is provided with a fulcrum 43 on which two pressure fluid supply pipes 4 intersect and become pivotable at the position of the branch pipe 41, and a handle 8, which is located between the fulcrum 43 and a coupling pipe 3 and includes an elastic body 44 such as a coil spring. The pressure fluid supply pipes 4 extend inside the handle 8. According to the second embodiment, since the elastic body 44 is used as the nozzle separation/approach means 6, nozzles 5 on the tips of the two pressure fluid supply pipes 4 are constantly biased in the direction facing each other. Thus, the pressure fluid supply pipe 4 may be a pipe that does not have a restoring force such as an elastic force. That is, in the first embodiment, the treatment is performed while gripping the two pressure fluid supply pipes 4 with the fingers in the direction for reducing the distance therebetween. On the other hand, in the second embodiment, the distance between the two pressure fluid supply pipes 4 is expanded by gripping the handle 8, and a part of the skin layer is pinched and held by an elastic force of the elastic body 44. Thus, the holding force when the fingers are removed from the handle 8 is continuously a constant pressure by the elastic force of the elastic body 44. In order to vary the holding force, the elastic force of the elastic body 44 may be made adjustable or the force of gripping the handle 44 may be increased or reduced.

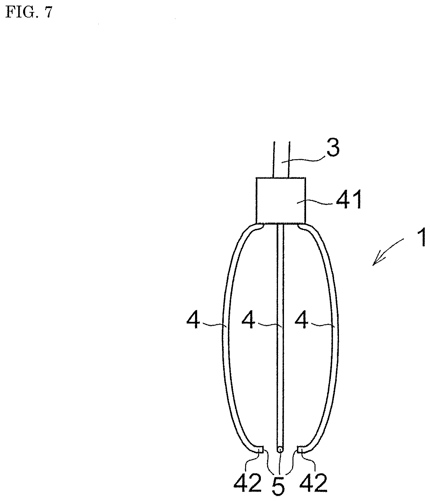

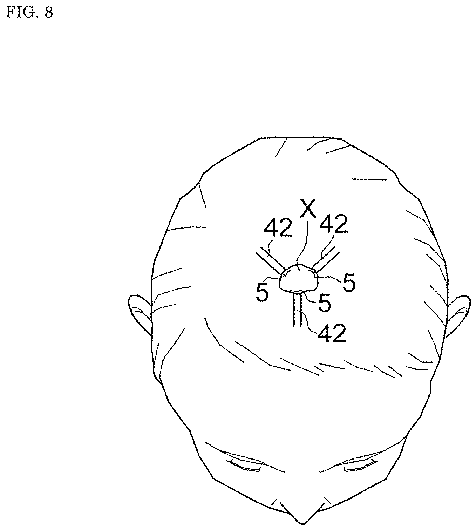

Fig. 7 illustrates a third embodiment of the present invention. The third embodiment differs from the first embodiment in that three pressure fluid supply pipes 4 extend from a branch pipe 41. The jet directions of the pressure fluid from nozzles 5 of the respective pressure fluid supply pipes 4 intersect at a point on the center. When a treatment is performed using these pressure fluid supply pipes 4, as illustrated in Fig. 8, the pressure fluid may be jetted from the three nozzles 5 with a part of the head where hair growth/hair restoration is to be promoted located on the central part between the three nozzles 5. Accordingly, the skin layer is raised by the pressure fluid from three sides. When the three pressure fluid supply pipes 4 are pushed with fingers toward the central part, the central part is raised to form a raised part X having a tent-like shape. Due to the same reason as the reason described in the first embodiment, coagulated fat cells under the skin layer inside the part surrounded on the three sides are split while making a sound, nutrients stored therein are released, and blood penetrates into the skin layer by the expansion and contraction of muscles, thereby supplying the nutrients to a hair root part. Accordingly, hair growth/hair restoration is promoted.

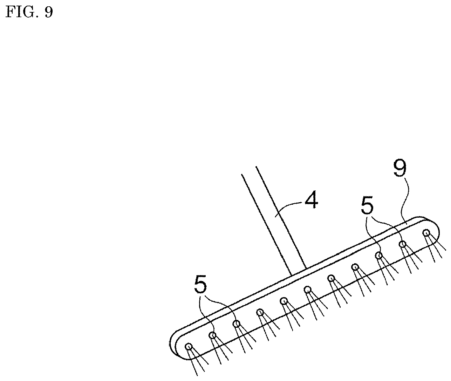

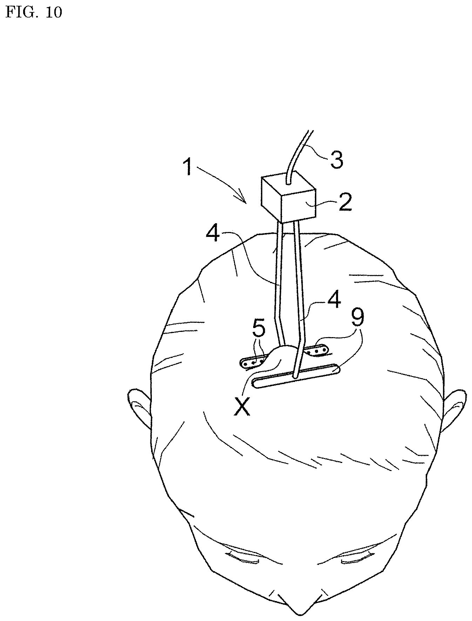

Fig. 9 illustrates a fourth embodiment of the present invention. The fourth embodiment is an example in which a large number of nozzles 5 are formed on the tip of a pressure fluid supply pipe 4. A branch pipe 9 is fixed to the tip of the pressure fluid supply pipe 4 perpendicularly to the pressure fluid supply pipe 4, and the branch pipe 9 communicates with the pressure fluid supply pipe 4. A large number of open holes are open on the branch pipe 9 to form the nozzles 5 on a face facing the other branch pipe 9 in a manner to be paired with and correspond to the respective open holes on the other branch pipe 9. The distance between the branch pipes 9 is 10 mm, which is approximately equal to the distance between the nozzles 5 of the first embodiment. When a treatment is performed using the hair growth/hair restoration promoting apparatus 1 of the present embodiment, as illustrated in Fig. 10, the two branch pipes 9 are put on the head to pinch a part where hair growth/hair restoration is to be promoted, and the distance between the pressure fluid supply pipes 4 is reduced while jetting the pressure fluid from the nozzles 5, thereby splitting the coagulated fat cells inside the skin layer of the formed long slender raised part X. The treatment is performed in a manner similar to the first embodiment. In the present embodiment, the treatment can be performed on a part having a large area at once due to the large number of nozzles 5. Instead of the large number of open holes as the nozzles 5, a long slender slit-like nozzle 5 formed in the lateral direction may be employed.

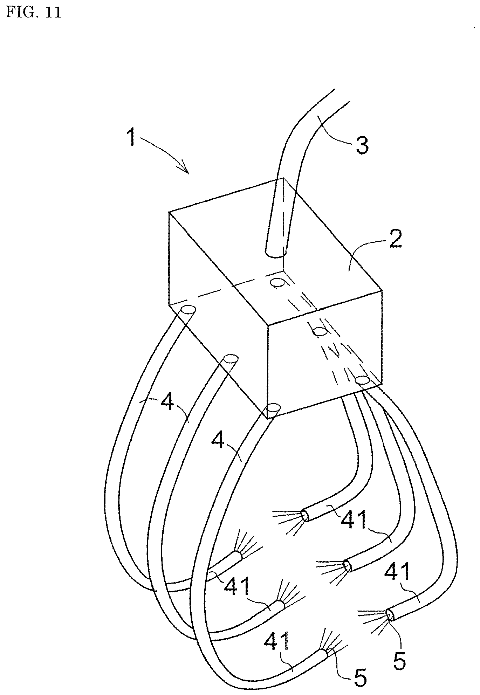

Fig. 11 illustrates a fifth embodiment of the present invention. In the fifth embodiment, three sets of pressure fluid supply pipes 4 of the first embodiment are disposed on a branch pipe 41. The other structure is the same as that of the structure of the first embodiment. When a treatment is performed, it is necessary to operate the six pressure fluid supply pipes 4 with fingers in the narrowing direction and the loosening direction. Thus, the pressure fluid supply pipes 4 on each side may be coupled with a coupler so that the pressure fluid supply pipes 4 can be operated at the same time. Also in the present embodiment, the nozzles 5 are arrayed in the lateral direction.
Thus, the treatment can be performed on a part having a large area by a single operation. Similarly to the above embodiment, since the large number of nozzles 5 are present, it is necessary to increase the supply amount of the pressure fluid from the pressure fluid supply source 2.

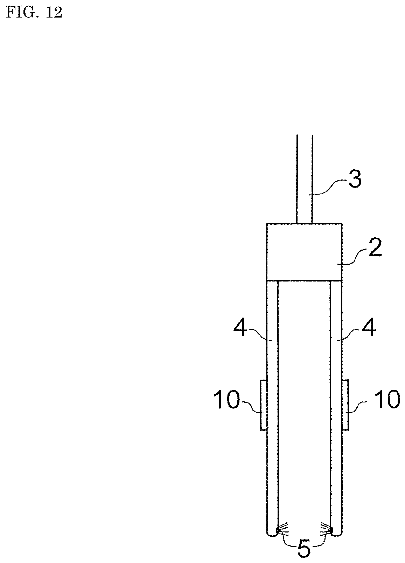

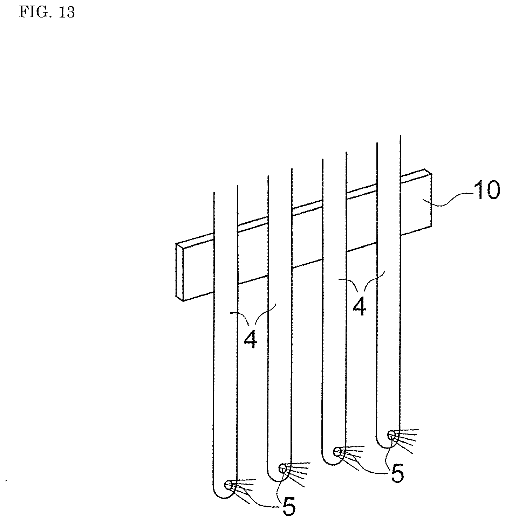

Figs. 12 and 13 illustrate a sixth embodiment of the present invention. The sixth embodiment differs from the first embodiment in that a plurality of pressure fluid supply pipes 4 which branch from a branch pipe 41 and face each other are coupled with a coupling plate 10 at each side, and open holes open on side faces of tips of the pressure fluid supply pipes 4 facing each other serve as nozzles 5 instead of forming the pinch pipes 42 by bending the tips of the pressure fluid supply pipes 4. Also in the present embodiment, in a manner similar to the fourth and fifth embodiments, a treatment can be performed on a part having a large area by a single operation due the large number of nozzles 5 which are paired and face each other. Further, the distances between all the paired nozzles 5 are reduced by merely reducing the distance between the coupling plates 10. Thus, the operation is easy. Further, since the pinch pipe parts 42 of the first embodiment are not provided, the distance between the two pressure fluid supply pipes 4 can be reduced, and the apparatus can thus be downsized. Further, when the position of a part to be treated is moved, hair is not caught on the apparatus. Thus, the operation efficiency is improved.

The above embodiments describe the case where the treatment is performed using gas, such as air, as the pressure fluid as illustrated in Figs. 2, 8, and 10. On the other hand, when the pressure fluid is liquid, such as water, the treatment may be performed on the head put above a washbowl in the same manner as washing hair or performed on the head in the same manner as washing hair by shower. Accordingly, even when no operator is present, a patient himself/herself can perform the treatment similarly to the case where the pressure fluid is gas. The water pressure is weak with only the pressure of tap water. Thus, water is preferably pressurized by a hydraulic pump or the like. In both the cases where the pressure fluid is gas and liquid, the shape of the open hole of the nozzle 5 is not limited to a circular shape, and may be another shape such as a horizontally oriented slit-like shape. It is possible to increase the jet pressure of the fluid within the bounds of not making the patient feel pain by reducing the open area of the nozzle. Accordingly, the treatment effect can be improved.

The above embodiments describe the hair growth/hair restoration on the head. However, hair growth/hair restoration can be promoted in a similar manner also in other parts of the body.

Further, in the above embodiments, the means using the restoring force of the pressure fluid supply pipes 4 or the biasing force of the elastic body 44 is described as the nozzle separation/approach means 6. Instead of this, the treatment may be performed in the following manner. When the pressure fluid from the pressure fluid supply source 2 is supplied by a switch operation in a state in which the nozzles 5 are put on a part where hair growth/hair restoration is to be promoted, the distance between the facing nozzles 5 which are separated from each other at a predetermined distance by the action of an elastic body or the like is reduced by the action of the pressure so that the skin layer is pinched. When the nozzles 5 are moved, the switch is turned off to expand the distance between the nozzles 5, the nozzles 5 are then moved to another part, and the switch is turned on again. These operations are continuously repeated.

Results of treatments performed using the method of the invention and the hair growth/hair restoration promoting apparatus will be described below. Treatment examples which were performed on six patients on the premise of confidentiality will be described below. The treatments were performed using the apparatus illustrated in Fig. 1 as the hair growth/hair restoration promoting apparatus 1 and the method as illustrated in Fig. 2 following the instructions described in [0029] and [0030].

### (Case 1)

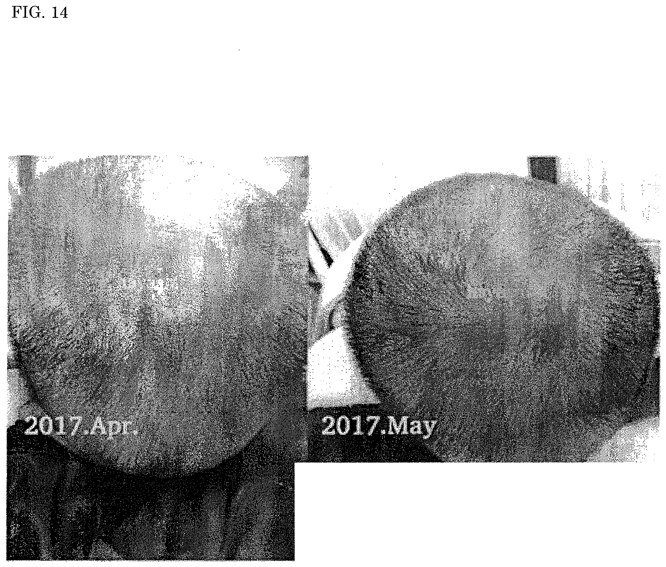

Patient: a male in his thirties
Frequency: once in two weeks
Time per one treatment: approximately 30 minutes
Target area: entire head
Duration: three times (three times in total)
Although no effect was obtained with an agent, thin hair started growing, and an effect was confirmed as a result of the treatment. The left side is before the treatment, and the right side is after the treatment (refer to Fig. 14).

### (Case 2)

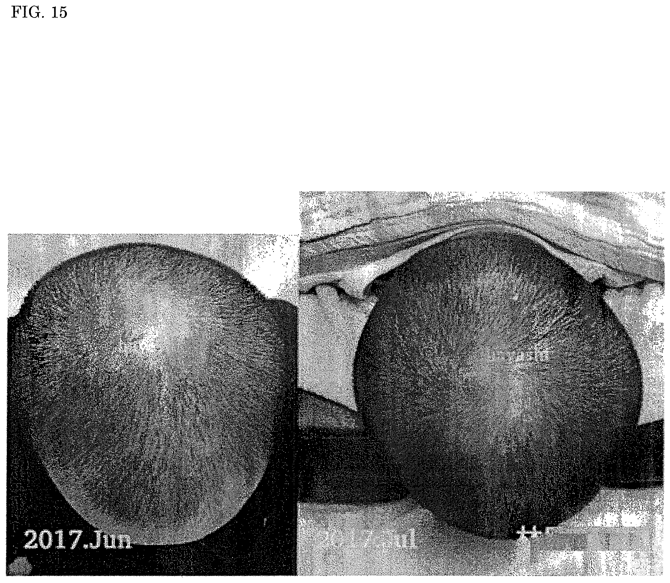

Patient: a male in his thirties
Frequency: once in two weeks
Time per one treatment: approximately 30 minutes
Target area: entire head
Duration: three times
As a result of the treatment, fine hair became thick, and multiple hairs grew from one pore. The left side is before the treatment, and the right side is after the treatment (refer to Fig. 15).

### (Case 3)

Patient: a male in his forties
Frequency: once in two weeks
Time per one treatment: approximately 30 minutes
Target area: entire head
Duration: two months
As a result of the treatment, transplanted own hair came off again. Then, the growth of multiple hairs from the pore was confirmed using a magnifier.

### (Case 4)

Patient: a female in her forties
Frequency: once in two weeks
Time per one treatment: approximately 30 minutes
Target area: entire head
Duration: four months
As a result of the treatment, thin hair after childbirth became thick, and the amount of hair also increased.

### (Case 5)

Patient: a female in her fifties
Frequency: once in two weeks
Time per one treatment: approximately 30 minutes
Target area: entire head
Duration: two months
As a result of the treatment, hair without volume became thick. Although hair used to be flat when leaving the office before the treatment, set hair can be maintained after the treatment.

### (Case 6)

Patient: a female in her sixties
Frequency: once in two weeks
Time per one treatment: approximately 30 minutes
Target area: entire head
Duration: four months
As a result of the treatment, thin hair, which hadn't been able to hide the skin even after setting the hair, became thick one by one, thin hair also started growing, and hair volume started increasing.

### INDUSTRIAL APPLICABILITY

The present invention is good news for people who are afflicted with thin hair. If many people wish to receive the treatment, the invigoration of the tertiary industry providing the treatment service is expected.

### LIST OF RERERENCE SIGNS

- 1: Hair growth/hair restoration promoting apparatus
- 2: Pressure fluid supply source
- 3: Coupling pipe
- 4: Pressure fluid supply pipe
- 41: Branch pipe
- 42: Pinch pipe part
- 43: Fulcrum
- 44: Elastic body
- 5: Nozzle
- 6: Nozzle separation/approach means
- 7: Band
- 8: Handle
- 9: Branch pipe
- 10: Coupling plate

## Claims

1. A treatment method for promoting hair growth/hair restoration comprising:
pinching a part of skin where hair growth/hair restoration is to be promoted and jetting a pressure fluid to the part from a periphery thereof to raise the part to burn fat under a skin layer in and near the part to which the pressure fluid is jetted to improve blood circulation to perform a treatment for promoting hair growth/hair restoration.

2. The treatment method for promoting hair growth/hair restoration according to claim 1, wherein the pressure fluid is air.

3. The treatment method for promoting hair growth/hair restoration according to claim 1, wherein the pressure fluid is water.

4. A hair growth/hair restoration promoting apparatus comprising:
a pressure fluid supply source;
a plurality of pressure fluid supply pipes configured to allow a pressure fluid supplied from the pressure fluid supply source to pass therethrough;
a plurality of nozzles formed on ends of the pressure fluid supply pipes at sides facing each other, the nozzles being configured to jet the pressure fluid; and
nozzle separation/approach means for causing the plurality of nozzles of the plurality of pressure fluid supply pipes to separate from and approach each other.

5. The hair growth/hair restoration promoting apparatus according to claim 4, wherein the pressure fluid is air.

6. The hair growth/hair restoration promoting apparatus according to claim 4, wherein the pressure fluid is water.

7. The hair growth/hair restoration promoting apparatus according to any one of claims 4 to 6, wherein the nozzle separation/approach means includes long slender pressure fluid supply pipes having a restoring force, the pressure fluid supply pipes being separable and approachable by an elastic force applied in an open direction of nozzles of the pressure fluid supply pipes or an opposite direction thereof.

8. The hair growth/hair restoration promoting apparatus according to any one of claims 4 to 7, wherein each of the nozzles includes an open hole open on a tip of a bent tip part of the pressure fluid supply pipe.

9. The hair growth/hair restoration promoting apparatus according to any one of claims 4 to 7, wherein each of the nozzles includes an open hole open on a side face of a tip part of the pressure fluid supply pipe.

10. The hair growth/hair restoration promoting apparatus according to any one of claims 4 to 7, wherein each of the nozzles includes a plurality of open holes open on a branch pipe disposed on a tip of the pressure fluid supply pipe so that the pressure fluid passes therethrough.
